# EUROPÄISCHE PATENTSCHRIFT

(11) **EP 0 283 819 B1**
(45) Veröffentlichungstag und Bekanntmachung des Hinweises auf die Patenterteilung: **17.08.1994**
(21) Anmeldenummer: 88103511.7
(22) Anmeldetag: 07.03.1988
(51) Int. Cl.: A61M 21/00

(54) **Einrichtung zur Durchführung von Hypnosetherapien**
Device to carry out a hypnosis-therapy
Dispositif pour effectuer une thérapie par hypnose

(30) Priorität: 24.03.1987 CH 1110/87
(43) Veröffentlichungstag der Anmeldung: 28.09.1988
(73) Patentinhaber: Bick, Claus, D-66994 Dahn (DE)
(72) Erfinder: Bick, Claus, D-66994 Dahn (DE)
(74) Vertreter: Petschner, Goetz

(56) Entgegenhaltungen:
- GB-A- 2 124 490
- US-A- 3 045 065
- US-A- 3 656 760
- US-A- 3 822 693

## Beschreibung

Die vorliegende Erfindung betrifft eine Einrichtung zur Durchführung von Hypnosetherapien, mit einer Mehrzahl, das Therapieren mehrerer Patienten gleichzeitig gestattende Kopfhörer-Stationen, welche wechselweise umschaltbar sind zwischen einem, allen Kopfhörer-Stationen gemeinsamen Steuerteil mit einer Tonwiedergabestufe zur gruppenmässigen Einleitung der Hypnose resp. Rückführung aus der Hypnose und je einem zugeordneten Tonträger-Abspielgerät zur Durchführung von Individualsuggestionen.

Psychogene Therapie-Verfahren in Form von Suggestiv-Behandlungen mit therapeutischem und prophylaktischem Charakter gewinnen immer mehr an Bedeutung, da hier eine sehr hohe Wahrscheinlichkeit einer positiven Beeinflussung des Unterbewusstseins besteht. Bei diesen Behandlungen werden in einem vertieften physischen und psychischen (hypnotischen) Entspannungszustand - welchem selbst schon eine erhebliche therapeutische Wirkung zukommt - suggestiv die negativen seelischen (Stör-) Faktoren, die im Unterbewusstsein haften und das Fehlverhalten (unbewusst) auslösen, durch positive seelische Antriebselemente ersetzt.

Trotz der Notwendigkeit individuell verschiedener Behandlungen zur Durchführung dieses vorerwähnten Austausches ist es zur Entlastung der behandelnden Therapeuten unumgänglich und möglich, zumindest die Einleitung, Aufrechterhaltung und Vertiefung des Entspannungszustandes mittels Apparatur durchzuführen.

Dies kann ansich mit einem Sprachlabor der vorgenannten Art, welches als Weiterentwicklung nach der US-A-3045065 gesehen werden kann, durchgeführt werden.

Es wurde nun aber gefunden, dass bestimmte Geräusche, wie insbesondere Meeresrauschen, in hohem Masse sedativ wirken.

Um das hier einsetzen zu können, ist erfindungsgemäss vorgesehen, dem gemeinsamen Steuerteil einen entsprechenden Rauscherzeuger zuzuordnen, der dann wahlweise einschaltbar und wechselweise umschaltbar auf eine Verstärker- und Modulationsstufe der Individual-Tonträger-Abspielgeräte resp. auf eine der Tonwiedergabestufe zur Gruppentherapie nachgeschaltete Modulationsstufe sein sollte, um das Meeresrauschen sowohl für sich alleine als auch den Gruppen- und Individualsuggestionen überlagert auf die Kopfhörer zu bringen.

Vorzugsweise ist dann dem Rauscherzeuger eine regelbare, zweckmässig zeitgesteuerte Schwellungsstufe nachgeschaltet, um die Lautstärke des Rauschens dem Ablauf der Therapie anpassen zu können.

Zudem können zusätzlich Echo- und/oder Hall-Stufen zuschaltbar sein.

Für einen zeitgesteuerten automatischen Ablauf einer Therapie-Sitzung ist es zudem vorgesehen, dem gemeinsamen Steuerteil einen Timer zuzuordnen, der eine Umschaltung von der gruppenmässigen Tonwiedergabestufe auf die Tonträger-Abspielgeräte der Stationen und zurück bewirkt. Beispielsweise kann der Timer eine erste Umschaltung nach Ablauf der Hypnose-Einleitung nach ca. 20 Minuten für ca. 5 Minuten auf Individualsuggestion und dann eine Rückschaltung für ca. 30 Minuten auf Gruppeninformation für Vertiefung der Hypnose und Rückführung aus der Hypnose vornehmen.

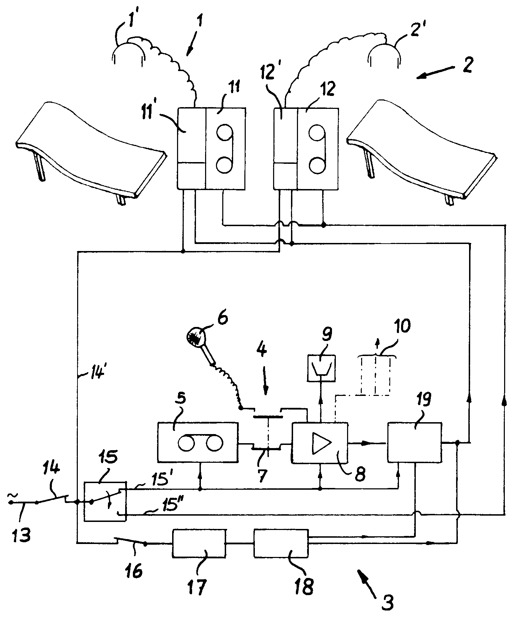

Eine beispielsweise Ausführungsform der erfindungsgemässen multifunktionalen Hypnose-Einrichtung ist nachfolgend anhand der Zeichnung, welche die Einrichtung in einem Funktionsschema veranschaulicht, näher erläutert.

Die gezeigte multifunktionale Hypnose-Einrichtung umfasst hier nur zwei Kopfhörer-Stationen 1 und 2 zum Therapieren von zwei Patienten gleichzeitig. Es sei aber betont, dass solche Einrichtungen für praktisch beliebig viele Patientenstationen ausgelegt werden können. In der Regel werden wohl 6 bis 20 Stationen vorzusehen sein.

Die Kopfhörer-Stationen 1 und 2, welche beispielsweise direkt an den Ruheplätzen für die Patienten angebaut sein können oder sich in Beistellmöbeln oder dgl. befinden, umfassen je einen Kopfhörer 1' resp. 2' sowie ein zugeordnetes Tonträger-Abspielgerät 11 und 12 mit einer zugeschalteten Verstärker- und Modulationsstufe 11' resp. 12'. Ferner können noch Lautsprechermittel (nicht gezeigt) vorgesehen sein.

Als Tonträger-Abspielgeräte können hier handelsübliche CD-Geräte oder Tonband-Geräte dienen. Ebenso sind die Kopfhörer, die Verstärker- und Modulationsstufen sowie die Lautsprechermittel von handelsüblicher Bauart und bedürfen so keiner näheren Erläuterung.

Die Kopfhörer-Stationen 1 und 2 resp. deren Kopfhörer 1' resp. 2' sind nun wechselweise umschaltbar zwischen deren Tonträger-Abspielgeräten 11 resp. 12 und einem, allen Kopfhörer-Stationen 1 und 2 gemeinsamen Steuerteil 3, der eine Tonwiedergabestufe 4 zur gruppenmässigen Einleitung der Hypnose resp. deren Vertiefung resp. der Rückführung aus der Hypnose umfasst.

Diese Umschaltung erfolgt zweckmässig über einen Timer 15, der wechselweise die Tonwiedergabestufe 4 über die Leitung 15' oder die individuellen Tonträger-Abspielgeräte 11 und 12 über die Leitung 15'' an das Netz 13 anschliesst, wobei dem Timer 15 natürlich ein Netzschalter 14 vorgeschaltet ist. Dieser Netzschalter 14 schliesst gleichzeitig auch die den Einzelmikrofonen 1' und 2' der Stationen vorgeschalteten Verstärker- und Modulationsstufen 11' resp. 12' über Leitung 14' an das Netz 13 an.

Die genannte Tonwiedergabestufe 4 zur Gruppentherapie umfasst hier ein Tonträger-Abspielgerät 5 üblicher Bauart sowie ein Mikrofon 6, die über Schaltermittel 7 wechselseitig einschaltbar sind.

Das genannte Mikrofon 6 und gegebenenfalls auch das Tonträger-Abspielgerät 5 sind wechselweise anschliessbar über geeignete Schaltermittel an der Verstärkerstufe 8 an einen Raum-Lautsprecher 9 oder an die Anschlüsse 10 der Verstärkerstufe 8 für die vorerwähnten (nicht gezeigten) Stationen-Lautsprecher bzw. direkt an die Kopfhörer 1' und 2' der Stationen 1 und 2.

Dem gemeinsamen Steuerteil 3 ist ferner ein Rauscherzeuger 17 zur Erzeugung von Meeresrauschen u. dgl. zugeordnet, der wahlweise über den Schalter 16 einschaltbar und durch den Timer wechselweise umschaltbar auf die Verstärker- und Modulationsstufen 11 und 12 der Stationen resp. Individual-Tonträger-Abspielgeräte 11 und 12 oder auf die Tonwiedergabestufe 4 zur Gruppentherapie resp. einer nachgeschalteten Modulationsstufe 19 ist. Vorzugsweise ist dabei dem Rauscherzeuger 17 eine regelbare, zeitgesteuerte Schwellungsstufe 18 nachgeschaltet.

Auf diese Weise besteht nun eine multifunktionale Hypnose-Einrichtung, die allen denkbaren Anforderungen zu genügen in der Lage ist und die sich durch Verwendung gebräuchlicher Geräte, Verstärker- und Modulationsstufen sowie Schalteinheiten leicht und kostengünstig aufbauen lässt. Dabei ist für beliebigen Stationen-Anschluss praktisch nur die Anschluss-Zahl und die Ausgangsleistung des Verstärkers der gruppenseitigen Tonwiedergabe des gemeinsamen Steuerteiles massgeblich. Weiter ist diese Einrichtung durch weitere Tonträger-Abspielgeräte für die zentrale Tonwiedergabestufe ergänzbar, etwa für Reserven oder auch für die Anwendung von Subliminal-Verfahren. Gleiches gilt auch bezüglich einer Erweiterung der Einrichtung durch lichterzeugende und lichtbeeinflussende sowie Hall oder Echo erzeugende Schaltungsanordnungen.

## Patentansprüche

1. Einrichtung zur Durchführung von Hypnosetherapien, mit einer Mehrzahl, das Therapieren mehrerer Patienten gleichzeitig gestattende Kopfhörer-Stationen (1,2,....), welche wechselweise umschaltbar sind zwischen einem, allen Kopfhörer-Stationen (1,2,...) gemeinsamen Steuerteil (3) mit einer Tonwiedergabestufe (4) zur gruppenmässigen Einleitung der Hypnose resp. Rückführung aus der Hypnose und je einem zugeordneten Tonträger-Abspielgerät (11,12,...) zur Durchführung von Individualsuggestionen, dadurch gekennzeichnet, dass dem gemeinsamen Steuerteil (3) ein Rauscherzeuger (17) zur Erzeugung von Meeresrauschen zugeordnet ist, der wahlweise einschaltbar und wechselweise umschaltbar auf eine Verstärker- und Modulationsstufe (11', 12',...) der Individual-Tonträger-Abspielgeräte (11,12,...) resp. auf eine der Tonwiedergabestufe (4) zur Gruppentherapie nachgeschalteten Modulationsstufe (19) ist.

2. Einrichtung nach Anspruch 1, dadurch gekennzeichnet, dass dem Rauscherzeuger (17) eine regelbare, zeitgesteuerte Schwellungsstufe (18) nachgeschaltet ist.

3. Einrichtung nach Anspruch 1, dadurch gekennzeichnet, dass dem gemeinsamen Steuerteil (3) ein Timer (15) zur zeitgesteuerten Umschaltung von der gruppenmässigen Tonwiedergabestufe (4) auf die Tonträger-Abspielgeräte (11,12...) der Stationen (1,2,...) und zurück zugeordnet ist.

## Claims

1. An apparatus for carrying out hypnotherapy, comprising a plurality of headset stations (1,2,...) enabling simultaneous treatment of a plurality of patients, which headset stations may alternately be switched between one control element (3) common to all headset stations (1,2,...), said control element (3) having a sound reproduction stage (4) for group inducement of hypnosis and recovery from hypnosis, and sound carrier play-back apparatuses (11,12,...) associated with each for carrying out individual suggestions, characterized in that a noise generator (17) for generating sea sounds is associated with the common control element (3), which noise generator (17) may be selectively switched on and alternately switched between an amplifying and modulation stage (11',12',...) of the individual sound carrier play-back apparatuses (11,12,...) and a modulation stage (19) connected downstream of the sound reproduction stage (4) for group therapy.

2. An apparatus according to claim 1, characterized in that an adjustable, timed swelling stage (18) is connected downstream of the noise generator (17).

3. An apparatus according to claim 1, characterized in that a timer (15) for timed switching from the group sound reproduction stage (4) to the sound carrier play-back apparatuses (11,12,...) of the stations (1,2,...) and back is associated with the common control element (3).

## Revendications

1. Dispositif pour effectuer des thérapies par hypnose avec plusieurs postes à casque-écouteur (1, 2,...) permettant le traitement simultané de plusieurs patients, lesquels postes sont commutables en alternance entre une partie de commande commune (3) à tous les postes à casque-écouteur (1, 2,...) présentant un étage de reproduction sonore (4) pour l'amorce de l'hypnose en groupe resp. pour le retour de l'hypnose et un appareil de lecture de support sonore (11, 12...) pour l'exécution de suggestions individuelles, caractérisé en ce que la partie de commande commune (3), pour produire le rugissement de la mer, est associée à un générateur de bruit (17) qui peut être mis en marche de manière sélective et être commuté en alternance sur un amplificateur et modulateur (11', 12',...) des appareils individuels de lecture de support sonore (11, 12,...) resp. sur un étage de modulation (19) intervenant à la suite de l'étage de reproduction sonore (4) destiné à la thérapie de groupe.

2. Dispositif selon la revendication 1, caractérisé en ce que l'étage de seuil (18) réglable à commande temporelle précède le générateur de bruit (17).

3. Dispositif selon la revendication 1, caractérisé en ce que la partie de commande commune (3) est associée à une minuterie (15) pour la commutation commandée temporellement entre l'étage de reproduction sonore (4) en groupe et les appareils de lecture de support sonore (11, 12,...) des postes (1, 2,...).
